# EUROPÄISCHE PATENTANMELDUNG

(11) **EP 0 553 461 A1**
(43) Veröffentlichungstag der Anmeldung: **04.08.1993**
(21) Anmeldenummer: 92121215.5
(22) Anmeldetag: 12.12.1992
(51) Int. Cl.: A61M 1/00

(54) **Spül- und Saugvorrichtung, insbesondere für die Chirurgie**

(30) Priorität: 12.12.1991 DE 4141014; 05.06.1992 DE 9207627 U
(71) Anmelder: ELEKTRONIK-VERTRIEB GmbH, D-97469 Gochsheim (DE)
(72) Erfinder: Krüger, Manfred, W-8726 Gochsheim (DE)
(74) Vertreter: Flosdorff, Jürgen, Dr.

(57) **Zusammenfassung**

Die Spül- und Saugvorrichtung enthält ein Handstück (46,30,26,23,10), an dem ein Spülschlauch (30',41) und ein Saugschlauch (31',39) angeschlossen werden, sowie eine rohrförmige Verlängerung (12,33), die einen Spülkanal und einen Saugkanal (17,24,28,18,25) enthält und in eine Operationswunde eingeführt wird. Der Saugkanal des Handstücks steht über eine Bypass-Bohrung (20) mit der umgebenden Atmosphäre in Verbindung. Durch Schließen der Bypass-Bohrung wird exakt dosiert Flüssigkeit abgesaugt. Zugeführt wird die Spülflüssigkeit mittels einer geräuschlos arbeitenden, stufenlos steuerbaren Rollerpumpe (4), die durch einen Fußschalter (5) gesteuert wird, der eine Feindosierung der Förderrate ermöglicht. Neben einer Volumensteuerung der Spülpumpe (4) kann auch eine Druckregelung vorgesehen sein, wozu die Spülflüssigkeitsleitung mit einer Spülklemme (54) versehen ist. Die Spül- und Saugvorrichtung ermöglicht eine optimale Flüssigkeitsbilanzierung in einer Operationswunde, wodurch eine Pfützenbildung oder ein Austrocknen des Gewebes verhindert ist. Die Vorrichtung kann von einem operierenden Chirurgen mit einer Hand betätigt werden. Durch den sanft an- und ablaufenden Spül- und Saugvorgang wird eine Schädigung von empfindlichem Gewebe zuverlässig vermieden.

## Beschreibung

Die Erfindung betrifft eine Spül- und Saugvorrichtung insbesondere für die Chirurgie, die bei operativen Eingriffen dem Einbringen physiologischer Lösungen zur Vermeidung eines Austrocknens freigelegter Gewebestelle, der Sekretbeseitigung zur Verbesserung der Operationsfeldübersicht und der wundgebietspflege dient. Die Spül- und Saugvorrichtung ist jedoch darüberhinaus allgemein zur Förderung der Heilung von Wunden einsetzbar, die beispielsweise eine Folge von Unfällen sind.

Bei operativen Eingriffen ist es häufig notwendig, zum Zwecke einer besseren Übersicht über die Wundverhältnisse die Wundflüssigkeiten, d.h. Blut und sonstige Sekrete, zu entfernen. Dies geschieht heute noch häufig mittels Abtupfen mittels Tupfer, durch die irreversible Gewebestörungen bzw. Gewebetraumatisationen hervorgerufen werden können. Außerdem besteht die Gefahr, daß solche Tupfer in der Operationswunde "vergessen" werden und dort zurückbleiben.

Bekannt ist auch das Absaugen von Flüssigkeiten mittels eines Vakuumsaugers, der mit einem sterilen Schlauchsystem verbunden ist, welches am Absaugort eingesetzt wird. Ein solcher Absauger kommt vor allem dann zum Einsatz, wenn physiologische Lösungen auf Anforderungen des Arztes in die Eingriffsstelle eingebracht werden, um diese freizuspülen und zu säubern und/oder um ein Austrocknen der freigelegten Gewebestellen zu verhindern.

Die physiologische Lösung wird häufig in einem offenen Gefäß vorgewärmt und auf Anforderung mittels eines manuellen Schöpfgefäßes eingebracht. Hierbei ist die erforderliche Sterilität der physiologischen Lösung nicht gewährleistet, und es ist nicht möglich, die eingebrachte Lösungsmenge fein zu dosieren. Dadurch kann ein Überlauf an der Operationsstelle entstehen, der zur Pfützenbildung und zu Feuchtigkeitsstraßen führt. Während einer Hochfrequenz-Chirurgie kann dies die Sicherheit wegen unkontrollierter Stromflüsse beeinträchtigen, und es kommt allgemein zu möglichen Kontaminationsbrücken.

Es ist auch bekannt, Flüssigkeiten mittels einer Kompressoreinheit einzubringen, die mit einem sterilen Schlauch verbunden ist, dessen am Eingriffsort einzusetzendes Ende mit einem manuell zu betätigen Ventil versehen ist. Auf Anforderung zum Spülen wird dieses Ventil freigegeben, wodurch die Spülflüssigkeit mit dem Überdruck der Kompressoreinheit in die Operationswunde eingebracht wird. Auch auf diese Weise läßt sich die Förderrate nicht einstellen, und der beträchtliche Förderdruck kann bei unsachgemäßer Handhabung zur Perforation oder aber zur Gewebetraumatisierung führen. Außerdem wird durch die Elastizität des Schlauchsystems eine bolusartige Entladung bewirkt, bei der eine Lösungsmenge mit verhältnismäßig hohem Druck aus dem Ventil herausschießt. Durch die Elastistizität des Schlauchsystems ist zudem eine zeitlich exakte Begrenzung des Flüssigkeitsstromes kaum mehr möglich.

Ein weiterer Nachteil des bekannten Flüssigkeitstransportes mittels Überdruck liegt darin, daß die Kompressoreinheit fortlaufend störende Geräusche entwickelt, die die Verständigung des Operationsteams beeinträchtigen können. Ein zusätzliches Sicherheitsrisiko ist durch das mögliche Bersten der Überdruckkammer oder des Schlauchsystems gegeben.

Der vorliegenden Erfindung liegt die Aufgabe zugrunde, eine Spül- und Saugvorrichtung insbesondere für die Chirurgie anzugeben, mit der die vorstehend beschriebenen Nachteile bekannter Vorrichtungen und Methoden vermieden sind.

Eine weitere Aufgabe der Erfindung besteht darin, eine Spül- und Saugvorrichtung anzugeben, bei der sowohl der Spülvorgng als auch der Saugvorgang von einer einzigen Person, d.h. im Falle einer Operation von dem Chirurgen gesteuert werden kann, dem hierzu nur eine Hand zur Verfügung steht.

Eine weitere Aufgabe der Erfindung besteht darin, eine simultan arbeitende Spül- und Saugvorrichtung anzugeben, die so arbeitet, daß der Spül- und der Saugvorgang ein äußerst empfindliches Gewebe nicht schädigt.

Eine weitere Aufgabe der Erfindung besteht darin, eine Spül- und Saugvorrichtung der betrachteten Art anzugeben, die eine einfache und sichere Flüssigkeitsbilanzierung ermöglicht, bei der die zugeführte Spülflüssigkeitsmenge und die abgesaugte Flüssigkeitsmenge genauestens aufeinander abgestimmt werden können. Eine Pfützenbildung oder ein Austrocknen des Gewebes soll mittels der erfindungsgmäßen Vorrichtung zuverlässig verhindert werden. Dabei soll der simultan ablaufende Spül- und Saugvorgang sowohl schnell als auch fein aufeinander abstimmbar sein, so daß eine der jeweiligen Situation des Operations- oder Wundgebiets entsprechende Flüssigkeitsbilanzierung erfolgen kann.

Diese Aufgabe wird erfindungsgemäß durch die im Kennzeichen des Anspruchs 1 angegebenen Merkmale gelöst. Vorteilhafte Weiterbildungen der Erfindung sind in den Unteransprüchen gekennzeichnet.

Die erfindungsgemäße Spül- und Saugvorrichtung enthält ein Handstück, welches der Benutzer zur Handhabung der Spül- und Saugvorrichtung in der Hand hält und welches einerseits mit einer Zuführleitung für eine Spülflüssigkeit und mit einer Saugleitung verbunden ist und andererseits eine vorzugsweise rohrförmige Verlängerung aufweist, in der sich ein Spül- und ein Saugkanal befinden, die gemeinsam in die Operationswunde eingeführt werden, um simultan oder auch nacheinander Spülflüssigkeit einzubringen und/oder Flüssigkeit abzusaugen.

In einer vorteilhaften Weiterbildung der Erfindung ist das Handstück ferner mit einer Einrichtung zum Hervorrufen eines Saugvorgangs versehen, welche in einer besonders bevorzugten Ausgestaltung durch eine Bypass-Leitung gebildet ist, die die Saugleitung des Handstücks mit der umgebenen Atmosphäre verbindet. Damit kann auf die Saugleitung, die sich in dem Handstück zu dem Saugkanal der rohrförmigen Verlängerung fortsetzt, stets ein Unterdruck einwirken, ohne daß dies unmittelbar zu einem Saugvorgang der in die Wunde eingeführten rohrförmigen Verlängerung des Handstücks führt, da durch die Bypass-Leitung solange Fremdluft angezogen wird, wie diese unverschlossen bleibt. Erst wenn die Bypass-Leitung des Handstücks verschlossen wird, was vorzugsweise manuell, d.h. durch einen Finger der Bedienungsperson, geschieht, wird Flüssigkeit vom Kopfende des Handstücks in die nachgeordnete Vakuumkammer abgezogen. Hierdurch kann der Saugvorgang schnell und einfach exakt gesteuert werden. Die Bypass-Leitung bzw. Bypass-Öffnung kann auch durch einen hierzu angeordneten Schieber oder dergleichen verschließbar sein.

Wenn die Bypass-Bohrung durch eine Wand des Handstücks verläuft, um den Saugkanal mit der äußeren Atmosphäre zu verbinden, ist sie zweckmäßigerweise im Bodenbereich einer Griffmulde ausgebildet, wo sie besonders sicher zu ertasten ist, so daß das Handstück ohne besonders Augenmerk der Bedienungsperson handhabbar ist.

Erfindungsgemäß ist ferner vorgesehen, daß die Zuführleitung mit wenigstens einer Rollerpumpe verbunden ist, deren Förderrate vorzugsweise mittels eines Fußschalters den jeweiligen Erfordernissen entsprechend steuerbar ist. Die Rollerpumpe arbeitet geräuschlos und mit verhältnismäßig geringem Druck, so daß Verletzungen des von der Spülflüssigkeit getroffenen Gewebes nicht zu befürchten sind. Die Förderrate der Pumpe ist stufenlos einstellbar.

Gemäß der Erfindung wird ferner vorgeschlagen, daß die physiologische Lösung in einem sterilen Beutel mittels einer Wärmeplatte auf einer Temperatur von 37o C gehalten wird, so daß es beim Spülvorgang zu keiner Traumatisierung infolge Temperaturdifferenzen kommen kann. Der Beutel wird mittels eines Anstichdorns mit dem sterilen Schlauchsystem konnektiert, welches als Einmal-Schlauchsystem nach einmaligem Einsatz ausgetauscht wird.

Es kann auch eine Doppelschlauchpumpe oder Doppelrollerpumpe vorgesehen sein, bei der wahlweise ein oder zwei Pumpensegmente mit einer entsprechenden Anzahl Lösungsbeutel verbunden ist bzw. sind.

Das Schlauchsystem besteht vorzugsweise aus einem Duoschlauch, d.h. zwei mechanisch miteinander fest verbundenen Schlauchleitungen, wodurch die Schlauchführung übersichtlich und die Knickfreiheit der Schläuche gewährleistet ist. Die Spülleitung kann zur Adaption an das Handstück einen Luer-Lock-Anschluß haben, während die Saugleitung handstückseitig vorzugsweise mittels eines Tüllenanschlusses befestigt ist. Der Saugschlauch kann selbstverständlich auch über einen Luer-Lock-Anschluß befestigt werden, während für den Spülschlauchanschluß auch eine Tülle vorgesehen sein kann.

Wie bereits erwähnt, wird der Spülvorgang vorzugsweise über einen Fußschalter gesteuert, wobei eine Feindosierung der Förderraten ermöglicht ist und der Bedienungsperson beide Hände für anderweitige Handhabungen zur Verfügung stehen. Der Absaugvorgang wird bevorzugt durch manuelles Öffnen bzw. Verschließen der Bypass-Bohrung durchgeführt und ist damit unmittelbar an die jeweiligen Verhältnisse im Wundgebiet anpaßbar und exakt steuerbar. Damit können exakt aufeinander abgestimmte Spül- und Saugvorgänge simultan ausgeführt werden, ohne daß die Gefahr der Verletzung des betroffenen Gewebes und einer Kontamination durch die Spül- und Saugvorgänge besteht. Zudem ist infolge des besonderen Handstücks und der Fußsteuerung der Pumpe keine zusätzliche Bedienungsperson erforderlich.

Die rohrförmige Verlängerung des Handstücks kann durch ein starres Rohr, gegebenenfalls durch zwei neben- oder übereinander angeordnete starre Rohre oder durch ein flexibles, schlauchähnliches Bauteil gebildet sein, wobei diese Verlängerung entweder einstückig mit dem Handstück ausgebildet oder z.B. an diesem angeschraubt sein kann.Wenn das Handstück mit der rohrartigen Verlängerung als Einmalartikel eingesetzt wird, besteht es vorteilhafterweise aus Polyurethan oder PVC.

Wenn die erfindungsgemäße Spül- und Saugvorrichtung für die Neurochirurgie verwendet wird, ist vorzugsweise ein langgestrecktes, abgewinkeltes Kapillarrohr vorgesehen, welches an ein metallenes Handstück angeschraubt sein kann und dessen Lumen-Innendurchmesser vorzugsweise 3,0 mm bis 5 mm beträgt.

Wie bereits erwähnt, ist die rohrförmige Verlängerung durch den Spülkanal und den Saugkanal gebildet, wobei die Anordnung so getroffen sein kann, daß sich einer der beiden Kanäle mittig in der rohrförmigen Verlängerung befindet und von dem anderen Kanal umgeben ist. Dabei kann der Spülkanal z. B. in Form eines Spülschlauches innenliegen, wie dies bei dem für die Neurochirurgie vorgesehenen Handstück bevorzugt ist. Die Anordnung kann auch umgekehrt getroffen sein, indem ein innenliegender Saugschlauch vorzugsweise konzentrisch von dem Spülkanal umgeben ist. Bei dieser Ausgestaltung kann ferner vorgesehen sein, daß der vordere Endbereich des Rohrstücks mit einer Vielzahl von Spülöffnungen versehen ist, wodurch ein sogenanntes " Brausehandstück" entsteht, bei dem die Spülflüssigkeit aus einer Vielzahl von Wandöffnungen austritt. Dies ermöglicht eine großflächige Spülung.

In einer alternativen Ausgestaltung der Erfindung können, wie bereits oben erwähnt ist, die beiden Kanäle auch neben- oder übereinander angeordnet und miteinander verklebt sein.

Die Kanäle der rohrförmigen Verlängerung des Handstücks können sich auf geeignete Weise durch das eigentliche Handstück fortsetzen, wobei selbstverständlich die erforderlichen Dichtungen anzuordnen sind, die die Spülleitung von der Saugleitung trennen und einen Austritt der Flüssigkeiten aus der Vorrichtung verhindern.

In diesem Zusammenhang kann vorgesehen sein, daß der Anschluß des Spülschlauchs über einen Luer-Lock-Adapter erfolgt, der mit einem Kapillarrohr einen entsprechenden Kanal des Handstücks durchgreift und an den Kapillarspülkanal der rohrförmigen Verlängerung dicht anschließt. Im übrigen können die die angeschlossenen Schläuche mit den Kanälen der rohrförmigen Verlängerung verbindenden, das Handstück durchgreifende Kanäle getrennt voneinander verlaufen oder sich zu einem gemeinsamen Kanal vereinigen, wobei im letzteren Falle eine zusätzliche innere Schlauchleitung vorzusehen ist.

In einer besonders einfachen Ausgestaltung der Erfindung ist das Handstück durch den rückwärtigen Abschnitt des Spülkanals und des Saugkanals gebildet, die im vorderen Abschnitt neben- oder übereinanderliegend miteinander verklebt sind und sich zur Bildung eines Handstücks verzweigen, um in einem geringen Abstand voneinander vorzugsweise parallel zueinander zu verlaufen. Dieser Abschnitt, in dem das Saugrohr und das Spülrohr seitlich voneinander beabstandet sind, dient als Handgriff der Vorrichtung, der ebenfalls bequem handhabbar ist.

Zusätzlich zu der Volumenregelung der Spülpumpe oder auch alternativ zu dieser kann die Spülpumpe mittels einer Spülklemme druckgeregelt werden. Während der Vorteil der Volumenregelung darin liegt, daß die Spülflüssigkeit mit einem sanften Anlauf eingebracht wird und auch weiterhin sanft zugeführt werden kann, kann in einigen chirurgischen Bereichen -vorzugsweise in der Allgemeinchirurgie- auch ein kurzer, hoher Spülfluß erforderlich sein. Gemäß der vorliegenden Erfindung wird dies folgendermaßen erreicht:
An der Spülflüssigkeitspumpe befindet sich ein Umschalter von Raten-auf Drucksteuerung. Druckseitig der Pumpe zweigt von der Spülflüssigkeitsleitung eine Druckableitung zu einem Druckmonitor ab. An dem Druckmonitor wird von der Bedienungsperson ein Druck-Sollwert eingestellt. Wenn die Spülklemme geschlossen ist und damit die Spülleitung verschließt, pumpt die Spülflüssigkeitspumpe, bis dieser Sollwert erreicht ist.

Wenn nun die Spülklemme geöffnet wird, erfolgt ein hoher Spülfluß, wobei der Druck rasch abgebaut wird. Die Pumpe versucht den Druck-Sollwert zu erreichen und fördert entsprechend mit einem sehr hohen Volumen. Beim Verschließen der Klemme reduziert sich die Drehzahl der Spülpumpe auf Null, wenn der Druck-Sollwert wiederum erreicht ist.

Es wird betont, daß sowohl bei volumengeregelter als auch bei druckgeregelter Spülflüssigkeitszufuhr mit und ohne Bypass-Bohrung gearbeitet werden kann.

Bei allen Ausführungsformen der Erfindung können die Spül- und Saugrohre (die weiter oben auch als "rohrförmige Verlängerung" bezeichnet sind) geradlinig oder gebogen sein. Die Spülenden können offen oder als Brauseenden ausgeführt sein.

Gemäß der Erfindung ist eine simultan arbeitende Spül- und Saugvorrichtung vorgesehen, mit der auf solche Weise Spülflüssigkeit in eine Wunde zugeführt und dort angesammelte bzw. sich dort ansammelnde Flüssigkeit so abgesaugt werden kann, daß eine Pfützenbildung oder ein Austrocknen des Gewebes zuverlässig verhindert werden kann. Dies ist dadurch möglich, daß der simultan oder nacheinander ablaufende Spül- und Saugvorgang sowohl schnell als auch fein aufeinander abgestimmt werden kann, so daß eine der jeweiligen Situation des Operations- oder Wundgebiets entsprechende Flüssigkeitsbilanzierung erfolgen kann.

Hierbei ist von besonderem Vorteil, daß sowohl der Spülvorgang als auch der Saugvorgang von einer einzigen Person, d.h. im Fall einer Operation von dem Chirurgen gesteuert werden kann, und zwar mit nur einer Hand.

Der Spülvorgang kann mit geringem Druck sanft anlaufen, so daß auch äußerst empfindliches Gewebe nicht beschädigt werden kann. Jede schlagartige oder bolusartige Entladung des Spülkanals kann vermieden werden.

Die Spülflüssigkeitszufuhr ist mittels eines Fußtasters auf die jeweils optimale Menge fein dosiert einstellbar, wobei auch der Saugvorgang mittels der Bypass-Bohrung so steuerbar ist, daß dieser keinerlei Gewebeschäden hervorrufen kann. Im Verlaufe des Saugvorgangs kann auf diese Weise die abgesaugte Flüssigkeitsmenge genauestens auf die zugeführte Spülflüssigkeitsmenge bzw. das im Wundbereich vorhandene Flüssigkeitvolumen abgestimmt werden.

Weitere Merkmale, Vorteile und Einzelheiten der Erfindung ergeben sich aus der nachfolgenden Beschreibung einiger bevorzugter Ausführungsformen. Dabei zeigen auf rein schematische Weise:
- Fig. 1: die Zuführleitung und Saugleitung mit zugehörigen Bauteilen in einer rein schematischen Darstellung;
- Fig. 2A, 2B und 2C: ein insbesondere für die Neurochirurgie geeignetes Handstück mit rohrförmiger Verlängerung, in einer Seitenansicht, einer Stirnansicht und in einem Längsscnnitt, der den Anschlußbereich wiedergibt;
- Fig. 3: ein Handstück für die Allgemeinchirurgie;
- Fig. 4: ein Brausehandstück;
- Fig. 5: eine weitere Ausführungsform des Handstücks mit einstückiger rohrförmiger Verlängerung;
- Fig. 6: ein mit dem Handstück gemäß Fig. 5 verbindbares Schlauchsystem und
- Fig. 7: eine weitere Ausführungsform der Erfindung;
- Fig. 8: eine weitere Ausführungsform der Erfindung mit Spülklemme und
- Fig. 9: schematische Darstellungen der Enden der Saug- und Spülröhrchen.

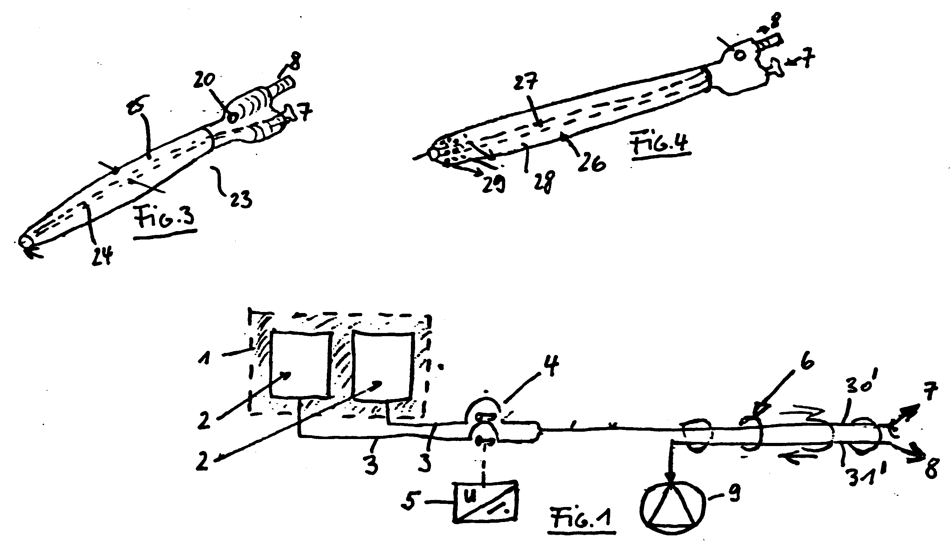

Fig. 1 zeigt auf rein schematische Weise eine Heizplatte 1, auf der zwei Kochsalzbeutel 2 angeordnet sind, die mittels nicht dargestellter Anstichdornen mit sterilen Schläuchen 3 in Verbindung stehen, die zu einer Doppelschlauchpumpe 4 mit wahlweise ein oder zwei Pumpsegmenten führen. Die Doppelschlauchpumpe bzw. Rollerpumpe 4 wird von einem Fußregler 5 gesteuert.

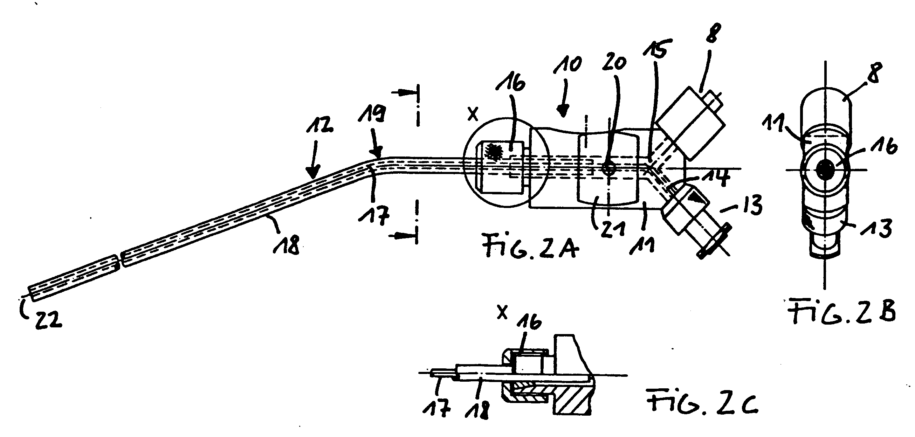

Ein Duoschlauch 6 führt mit einem mit den Schläuchen 3 verbundenen Spülschlauch zu einem Spülanschluß 7 eines in den Figuren 2 bis 5 dargestellten Handstücks, während der andere Schlauch des Duoschlauchs einen Vakuumsauger 9 mit einem Sauganschluß 8 des Handstücks verbindet.

Die Figuren 2A bis 2C zeigen ein Handstück 10, welches insbesondere für die Neurochirurgie geeignet ist. Das Handstück besteht aus dem eigentlichen Handstückteil 11 und einem mittels einer Überwurfmutter 16 daran angeschraubten Kapillarrohr 12. Der Spülschlauch ist an einen Luer-Lock-Adapter 13 angeschlossen, der sich in einem Kapillarrohr bzw. Schlauch 14 fortsetzt, der einen insgesamt X-förmigen Kanal 15 des Handstück 11 durchgreift und über eine geeignete Anschlußeinrichtung mit dem innenliegenden Spülkanal 17 der rohrförmigen Verlängerung 12 in Verbindung steht. Der innenliegende Spülkanal 17 ist von einem Saugkanal 18 im wesentlichen konzentrisch umgeben, der durch die Wand der rohrförmigen Verlängerung, d.h. des Kapillarrohres 12 begrenzt ist. Das Kapillarrohr 12 ist bei 19 abgewinkelt, um die Handhabbarkeit des Handstücks 10 zu verbessern.

Die Saugleitung des Duoschlauchs 6 ist ebenfalls über einen Luer-Lock-Anschluß (bei 8) mit dem Handstück 11 verbunden und steht über den insgesamt Y-förmigen Kanal 15 mit dem vorderen Saugkanal 18 in Verbindung. Eine Bypass-Bohrung 20 durchgreift die Wand des Handstückteils 11 und verbindet den Saugkanal mit der umgebenden Atmosphäre. Die Bypass-Bohrung 20 befindet sich im Bereich einer Griffmulde 21 des Handstück 11.

Während des Einsatzes des Handstücks 10 erzeugt der Vakuumsauger 9 ständig einen Unterdruck, der solange Fremdluft anzieht, wie die Bypass-Bohrung geöffnet bleibt. Wenn eine Bedienungsperson die Bypass-Öffnung verschließt, wird durch die vordere Öffnung 22 des Kapillarrohres 12 Flüssigkeit in den Kanal 18 eingesaugt und durch die weitere Saugleitung in einen mit dem Vakuumsauger 9 verbundenen Flüssigkeitsbeutel befördert. Dieser Saugvorgang kann bei gleichzeitiger Zufuhr von Spülflüssigkeit durch den Spülkanal 17 erfolgen.

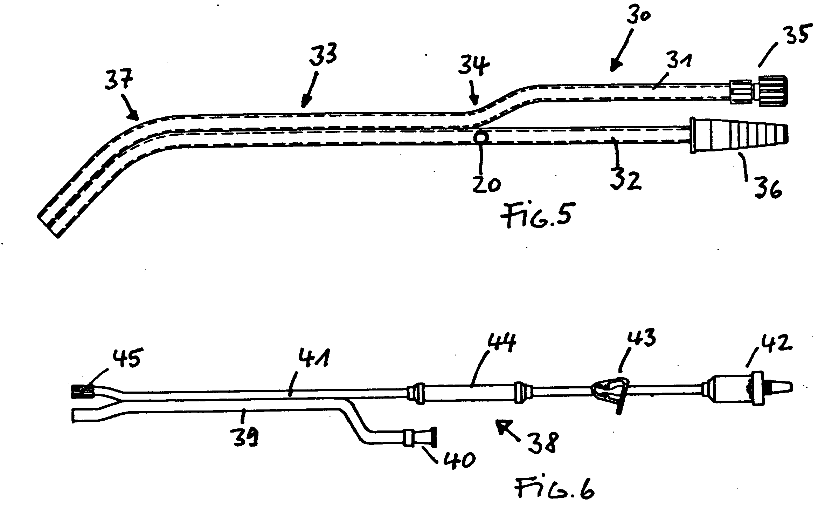

Fig. 3 zeigt ein Handstück 23, welches einstückig als Einmalartikel ausgebildet ist. Auch in dieser Ausführungsform liegt der Spülkanal 24 innen und ist durch einen zum Anschluß 7 führenden dünnen Schlauch gebildet. Der außen liegende Saugkanal 25 steht mit dem Anschluß 8 in Verbindung, außerdem über die Bypass-Bohrung 20 mit der umgebenden Atmosphäre.

Fig. 4 zeigt ein sogenanntes Brausehandstück 26, bei dem im Gegensatz zu den vorherigen Ausführungsformen der Saugkanal innen in der rohrförmigen Verlängerung liegt. Der außenliegende Spülkanal ist über eine Vielzahl von Spülöffnungen 29 in der Wand der rohrförmigen Verlängerung nach außen geöffnet, wodurch eine Wunde großflächig gespült und gereinigt werden kann. Das Brausehandstück 26 kann wiederum als Einmalartikel einstückig ausgebildet sein und beispielsweise aus Polyurethan oder PVC bestehen, oder aber es kann nach entsprechender Desinfektion wiederverwendbar sein, wobei dann zweckmäßigerweise die rohrförmige Verlängerung von dem rückwärtigen Handstückteil trennbar sein sollte.

Fig. 5 zeigt eine weitere bevorzugte Ausführungsform der Erfindung. Hierbei ist das Handstück 30 durch die rückwärtigen Abschnitte eines Spülrohrs 31 und eines Saugrohrs 32 gebildet, die im Bereich der rohrförmigen Verlängerung 33 neben bzw. übereinander liegen und miteinander verklebt sind und sich zur Bildung des Handstücks 30 bei 34 verzweigen, indem das Spülrohr 31 zunächst in einem Winkel von etwa 25o abgewinkelt verläuft, um dann in einem Abstand von ca. 10 mm parallel zum rückwärtigen Abschnitt des Saugrohrs 32 zu verlaufen. Das Spülrohr 31 ist zum Anschluß des Spülschlauchs mit einem weiblichen Luer-Lock-Anschluß 35 versehen, während das Saugrohr mit einer Saugtülle 36 zum Anschluß des Saugschlauches ausgerüstet ist.

Das Saugrohr 32 weist im Bereich der Abwinklung 34 des Spülrohrs 31 wiederum eine Bypass-Bohrung 20 auf, die verschlossen wird, wenn ein Saugvorgang eingeleitet werden soll.

Auch bei dieser bevorzugten Ausführungsform der Erfindung ist der vordere Bereich der rohrförmigen Verlängerung 33 bei 37 abgewinkelt, um die Handhabbarkeit der Vorrichtung zu verbessern. Der Winkel beträgt etwa 45o.

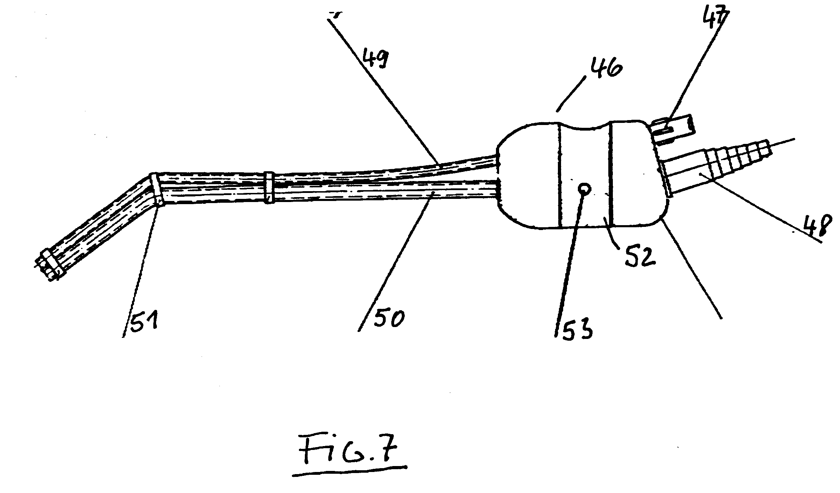

Fig. 6 zeigt ein Schlauchsystem 38, welches an das Handstück 30 der Ausführungsform gemäß Fig. 5 anschließbar ist.

Das Schlauchsystem 38 enthält eine Saugleitung 39, die einenends mit einem Katheteransatz 40 versehen ist und mit ihrem anderen Ende an die Saugtülle 36 des Saugrohres 32 anschließbar ist. Die Spülleitung bzw. der Spülschlauch 41 enthält eine Tropfkammer 42 mit Einstechteil, eine Handklemme 43, ein Pumpsegment 44 sowie an seinem in der Figur unteren Ende einen männlichen Luer-Lock-Anschluß 45, mit dem die Verbindung zu dem Luer-Lock-Anschluß 35 des Spülrohres 31 herstellbar ist.

Die beiden Schläuche 39 und 41 sind über einen großen Bereich aneinander angeklebt, wodurch ihre Knickfreiheit gewährleistet ist.

Die rohrförmige Verlängerung des Handstücks kann -wie oben erwähnt ist- ein doppellumiges Rohr sein, wobei bei Anwendung des Handstücks in der Allgemeinchirurgie der Lumeninnendurchmesser ca. 6mm bis 15 mm betragen sollte, während er bei der Anwendung des Handstücks in der Neurochirurgie etwa 3,0 mm bis 5 mm groß sein sollte.

Im Bereich des Handstücks ist vorzugsweise eine Bypass-Öffnung in der Saugleitung bzw. dem Saugrohr vorgesehen, um durch deren Verschluß einen Saugvorgang auszulösen. Es liegt jedoch im Rahmen der Erfindung, das Handstück ohne derartige Bypass-Bohrungen auszugestalten, so daß das Saugrohr ständig einen Saugvorgang ausführt.

Die in Figur 7 dargestellte Ausführungsform der Erfindung enthält ein Handstück bzw. einen Griffkörper 46, welcher an der in der Figur rechten Seite mit einem Spülanschluß 47 und einem Sauganschluß 48 versehen ist, während sich von seiner linken Seite ein oberes Spülrohr 49 und ein unteres Saugrohr 50 erstrecken, die mittels voneinander beabstandeter Sicherungseinrichtungen 51 zusammengehalten sind. Das Spülrohr 49 und das Saugrohr 50 sind im vorderen Endbereich abgewinkelt. In einer Griffmulde 52 des Griffkörpers 46 befindet sich eine Bypassbohrung 53, die in einer alternativen Ausführungsform auch entfallen kann.

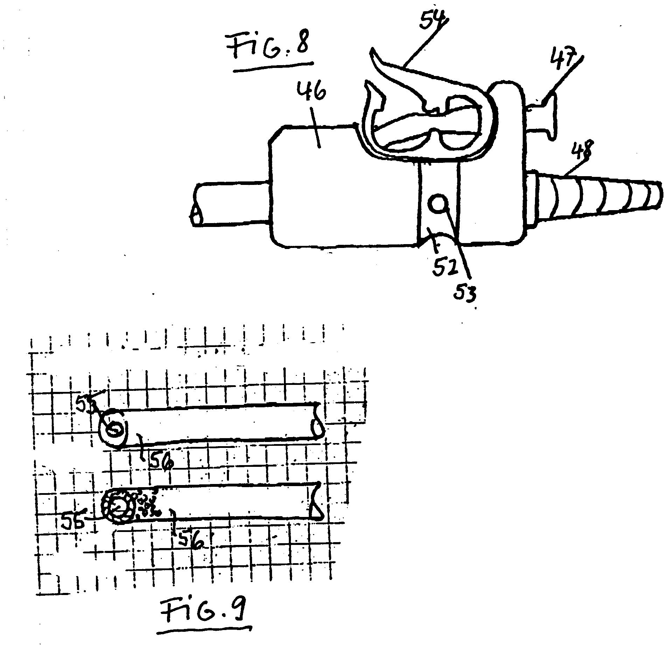

Fig. 8 zeigt eine weitere Ausführungsform der Erfindung, bei der einige Bestandteile mit denselben Bezugszeichen gekennzeichnet sind, wie bei der Ausführungsform gemäß Fig. 7. Ein wesentlicher Unterschied zu der vorstehend beschriebenen Ausführungsform besteht darin, daß eine Spülklemme 54 angeordnet ist, mit der ein durch den Griffkörper führender Spülschlauch verschlossen oder (gemäß Fig. 8) freigegeben werden kann. Die Spülklemme bzw. Schlauchklemme ist im wesentlichen formschlüssig in einer entsprechenden Aussparung des Griffkörpers angeordnet, so daß sie die Handhabung der erfindungsgemäßen Vorrichtung nicht beeinträchtigt. Bei dieser Ausführungsform befindet sich das vordere Spülrohr innerhalb des Saugrohres, wobei es im Rahmen der Erfindung liegt, die Anordnung umgekehrt zu treffen oder die beiden Rohre übereinander oder nebeneinander anzuordnen.

Fig. 9 zeigt schematisch den Endabschnitt einer "Rohr-in-Rohr-Anordnung" mit innenliegenden Saugrohr 55 und äußerem Spülrohr 56. Während bei der oberen Darstellung der Ringraum zwischen dem Saugrohr 55 und dem Spülrohr 56 nach außen offen ist, ist dieser bei der unteren Ausführungsform stirnseitig verschlossen, so daß die Spülflüssigkeit aus den zahlreichen kleinen Löchern im vorderen Endbereich des Spülrohres 56 nach Art einer Brause austritt.

Es wird darauf hingewiesen, daß beispielsweise auch die Ausführungsform gemäß Fig. 7 mit einer Spülklemme versehen werden kann, um hiermit zusätzlich oder alternativ zu einer Volumenregelung der Spülpumpe eine Druckregelung vorzusehen. Mit der Spülklemme kann die Spülleitung z.B. auch verschlossen werden, wenn die Pumpe mittels einer Raste auf eine feste Förderrate eingestellt ist und der Chirurg seinen Standort ändern will.

Außer für die genannten Fachgebiete der Chirurgie ist das erfindungsgemäße Handstück auch zum Einsatz in der Unfallchirurgie, Hals-Nasen-Ohren-Chirurgie, Gynäkologie und in der Zahnmedizin einsetzbar. Bei einfacher Handhabung lassen sich auf all diesen Gebieten alle Erfordernisse der Operationsfeldsäuberung und der Wundpflege durchführen.

## Patentansprüche

1. Spül- und Saugvorrichtung insbesondere für die Chirurgie,
**dadurch gekennzeichnet**,
daß eine Zuführleitung (30', 41) für eine Spülflüssigkeit und eine mit einer Vakuumquelle (9) verbundene Saugleitung (31', 39) an ein Handstück (10, 23, 26, 30, 46) anschließbar sind, welches eine in eine Operationswunde oder dergleichen einführbare, vorzugsweise rohrförmige Verlängerung (12, 33) aufweist, die sowohl einen Spül- als auch einen Saugkanal (17, 24, 28; 18, 25, 27; 31, 32, 49, 50) enthält.

2. Vorrichtung nach Anspruch 1,
dadurch gekennzeichnet, daß das Handstück eine Spülleitung und eine Saugleitung aufweist, wobei die letztere über eine Bypass-Leitung (20, 53) mit der umgebenden Atmosphäre in Verbindung steht, die durch eine Bohrung (20) durch eine Wand des Handstücks gebildet ist.

3. Vorrichtung nach der Anspruch 1,
dadurch gekennzeichnet, daß die Zuführleitung (30', 41) mit wenigstens einer Rollerpumpe (4) verbunden ist, zu deren stufenloser Steuerung und Drehzahlregelung ein Fußschalter (5) angeordnet ist.

4. Vorrichtung nach einem der Ansprüche 1 bis 3,
dadurch gekennzeichnet, daß die rohrförmige Verlängerung einen inneren Kanal (17, 24, 27) kleineren Durchmessers aufweist, der von dem durch die Wand der rohrförmigen Verlängerung begrenzten äußeren Kanal (18, 25, 28) umgeben ist.

5. Vorrichtung nach Anspruch 4,
dadurch gekennzeichnet, daß der innere Kanal der Saugkanal (27) ist.

6. Vorrichtung nach Anspruch 5,
dadurch gekennzeichnet, daß im vorderen Endabschnitt des Rohres eine Vielzahl von Spüllöchern (29) ausgebildet ist.

7. Vorrichtung nach einem der Ansprüche 1 bis 4,
dadurch gekennzeichnet, daß der innere Kanal der Spülkanal (17, 24) ist.

8. Vorrichtung nach einem der Ansprüche 1 bis 3,
dadurch gekennzeichnet, daß das Handstück (30) durch einen Abschnitt des Spülkanals (31) und des Saugkanals (32) gebildet ist und daß der Abschnitt des Spülkanals (31) und der Abschnitt des Saugkanals (32) zur Bildung des Handstücks (30) seitlich Voneinander beabstandet sind.

9. Vorrichtung nach einem der Ansprüche 1 bis 3 und 8,
dadurch gekennzeichnet, daß der Spülkanal (31) und der Saugkanal (32) der rohrförmigen Verlängerung (33) des Handstücks (30) nebeneinander oder übereinander liegend angeordnet sind.

10. Vorrichtung nach einem der Ansprüche 1 bis 9,
dadurch gekennzeichnet, daß die Spülleitung mit einer Spülklemme (54) versehen ist, mit der die Spülleitung wahlweise verschlossen oder freigegeben werden kann.
